Europäisches Patentamt

European Patent Office

Office européen des brevets

(11) Publication number: **0 217 055**
**B1**

(12) **EUROPEAN PATENT SPECIFICATION**

(45) Date of publication of patent specification: **09.01.91**

(21) Application number: **86110528.6**

(22) Date of filing: **30.07.86**

(51) Int. Cl.⁵: **A 61 M 1/00, A 61 M 5/00**

(54) A connector for plasmapheresis bag.

(30) Priority: **31.07.85 JP 167650/85**
**30.10.85 JP 241376/85**
**23.10.85 JP 161328/85 u**
**09.05.86 JP 68838/86 u**

(43) Date of publication of application:
**08.04.87 Bulletin 87/15**

(45) Publication of the grant of the patent:
**09.01.91 Bulletin 91/02**

(84) Designated Contracting States:
**DE FR GB**

(56) References cited:
**EP-A-0 063 333**
**WO-A-81/00053**
**US-A-3 394 954**
**US-A-3 625 212**
**US-A-3 902 489**
**US-A-3 945 380**
**US-A-4 076 285**
**US-A-4 150 673**

(73) Proprietor: **KAWASUMI LABORATORIES, INC.**
**No. 28-15, Minamioi, 3-chome Shinagawa-ku**
**Tokyo (JP)**

(72) Inventor: **Kawano, Yukihiro**
**No. 17-29, Deiki, 1-chome Kanazawa-ku**
**Yokohama-shi Kanagawa-ken (JP)**
Inventor: **Juji, Tomishiro**
**No. 9-12, Kikukawa, 2-chome**
**Sumida-ku Tokyo (JP)**
Inventor: **Ono, Toshihiko c/o Kawasumi Lab.Inc.**
**Saiki Kojo No. 1077, Oaza-Koda Yayoi-cho**
**Minamiamabe-gun Oita-ken (JP)**
Inventor: **Iga, Hirohumi c/o Kawasumi Lab.Inc.**
**Saiki Kojo No. 1077, Oaza-Koda Yayoi-cho**
**Minamiamabe-gun Oita-ken (JP)**
Inventor: **Ono, Seiichi c/o Kawasumi Lab.Inc.**
**Yayoi Kojo No. 2051, Oaza-Osakamoto Yayoi-cho**
**Minamiamabe-gun Oita-ken (JP)**
Inventor: **Isobe, Yoshiyuki c/o Kawasumi Lab.Inc.**
**Yayoi Kojo No. 2051, Oaza-Osakamoto Yayoi-cho**
**Minamiamabe-gun Oita-ken (JP)**
Inventor: **Watanabe, Michihiro**
**No.1796-2, Oaza-Kadota Yayoi-cho**
**Minamiamabe-gun Oita-ken (JP)**

Courier Press, Leamington Spa, England.

**EP  0 217 055  B1**

(74) Representative: **von Bezold, Dieter, Dr. et al
Dr. Dieter von Bezold Dipl.-Ing. Peter Schütz
Dipl.-Ing. Wolfgang Heusler Brienner Strasse 52
D-8000 München 2 (DE)**

## Description

Background of the invention
Field of the invention

The present invention relates to a connector for plasmapheresis bag set which is used for exsanguinating or drawing the blood from a blood donor, dividing a blood component and a blood plasma component, and after having stored the blood plasma component only, restoring a rest blood component to the blood donor.

Description of the prior art

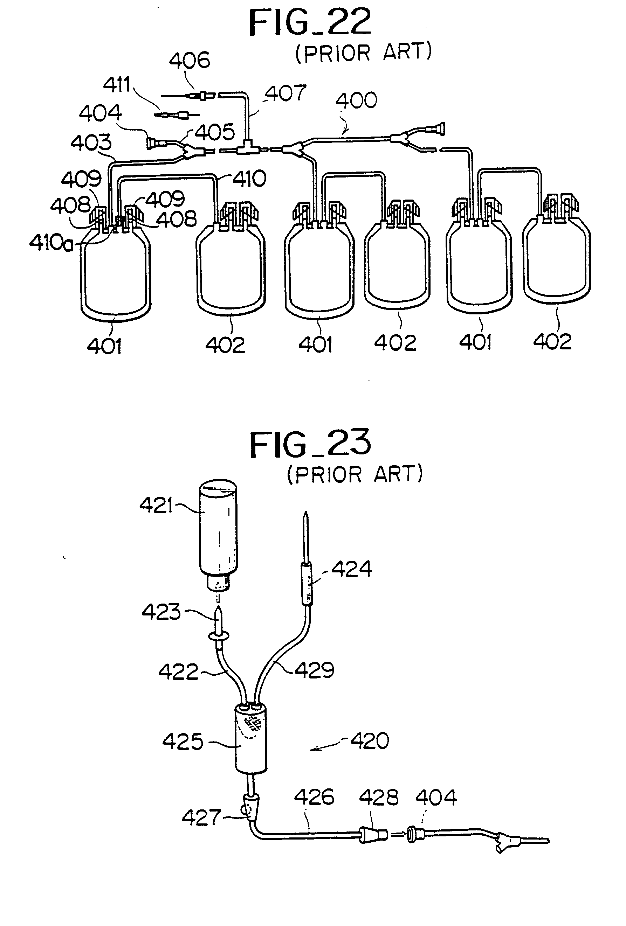

Fig. 22 shows an outlined view of a conventional plasmapheresis bag.

A plasmapheresis bag 400 comprises a pair of a blood exsanguinating bag 401 and a blood plasma dividing bag 402, and, if required, comprises a combination of two or three pairs.

The bag 401 is connected at its top to a blood drawing tube 403 which communicates with a blood restoring tube 405 having an exit 404 and a branched blood exsanguinating tube 407 having a needle 406.

Said bag 401 is furnished with a blood transfusing mouth 408 and a protector 409 therefor, and is connected to a tube 410 communicating with the blood plasma dividing bag 402 via a communication piece 410a.

In the prior art, when the above mentioned plasmapheresis bag 400 was composed of only a pair of the blood bag 401 and the blood plasma separating bag 402, it has been used as mentioned under.

The blood drawing needle 406 is pierced into a blood tube of the blood donor, and the blood is introduced into the blood bag 401 via the tubes 407 and 403. When a determined amount of the blood is gathered, the needle 406 is covered with the cap 411, and the tube 403 is closed with a welder and cut off. The bag 401 supporting the blood and the blood plasma separating bag 402 connected to the blood bag 401 are subjected to a centrifugal machine for dividing the blood in the bag 401 into the blood component and the blood plasma component.

Subsequently, the communication piece 410a is broken to open a flowing path, and a supernatant blood plasma component only is adopted in the blood separating bag 402 via the communicating bag 410, while the blood component remaining in the bag 401 is restored to the blood donor through a blood transfusion set 420 as shown in Fig. 23.

The blood transfusion set 420 is composed of a bottle 421 supporting a physiological saline solution containing an anti-coagulation agent, an introduction tube 422, a blood restoring needle 424, an instillation tube 425, a blood restoring tube 426, a clamp 427, and an adapter 428 provided at an end of said tube 426 and fittable to said exit 404.

For use, the saline solution is in advance filled in the blood restoring tube 426 of the blood transfusion set 420, the saline solution introducing tube 422 and the instillation tube 425. The adapter 428 is connected to the blood returning exit 404 and the needle 406 is again pierced into the blood tube of the blood donor, and a blood restoring needle 424 is passed through the blood transfusion mouth 408 of the bag 401. Subsequently, the blood component in the bag 401 is introduced to the instillation tube 425 via the tube 429, and is diluted by the saline solution in the instillation tube 425 and restored to the blood donor via the tubes 426, 405 and 407.

When the plasmapheresis bag set 400 is used, it must be confirmed that the blood component to be restored to the blood donor is the same as his.

The confirmation therefor has been carried out in the conventional manners as follows.

(1) Checking the blood donor's name and the name of the label of the blood exsanguination bag 401,
(2) Checking the number given to the blood donor and the number of the label of the blood bag 401,
(3) Checking the blood donor and the label attached to the blood bag 401.

However, since nowadays the blood plasma producing agents are much required and the blood donors of several hundreds is sealed with a day, human careless mistakes of confusing the names or numbers, or attaching places of the labels might be caused during working to invite vital troubles of the blood transfusion.

From US—A—4 076 285 is known a connector for coupling conduits in medical devices comprising a male connecting member and a female connecting member. The male connecting member is provided with a ring-like element formed with ingaging portions, and the female connecting member is provided with a ring-like element formed with complementary engaging portions in order to provide for a bayonet-like connection.

Summary of the invention

In the process that the blood exsanguination bag once separated from the plasmapheresis bag set is undertake with the centrifugal separation, and the blood plasma component is obtained, after which, said bag is once connected to said set, and for such a case that the blood component is returned to the blood donor, the present invention is to provide a connector which may exactly connect the separated exsanguinating bag to the initial plasmaphersis bag set, so that erroneous accidents between the blood donor and the blood component to be restored are avoided without fail.

A second object of the invention is to provide a connector which can be easily connected without requiring special technique, and keep blood running pathes hygienical.

A third object of the invention is to provide a compact connector which is easily set up and is produced at low cost.

The above mentioned object of the invention will be accomplished by an under mentioned connector for the plasmapheresis bag.

The connector of the invention comprises a first connecting member and a second connecting member, and these connecting members each are provided with a plurality of coaxially assembled key rings respectively, and the key rings are defined, at ends thereof, with engaging portions comprising grooves and projections, and are provided with positioning means. When said both members are connected the projections and grooves of the respective members are positioned to coincide each other only for one single connector.

Brief description of the drawings

Fig. 1 is a perspective view of a disassembled connector according to the invention;

Fig. 2 is a front view showing an upper half part of the connector in cross section;

Fig. 3A is a front view showing an upper half part of a housing of a male member in cross section;

Fig. 3B is a right side view of the housing of the male member;

Fig. 3C is a left side view of the housing of the male member;

Fig. 4A is a front view showing an upper half part of a housing of a female member in cross section;

Fig. 4B is a right side view of the housing of the female member;

Fig. 4C is a left side view of the housing of the female member;

Fig. 5A is a front veiw of a lower half part of a key ring of large diameter in cross section;

Fig. 5B is a right side view of the large diametered key ring;

Fig. 5C is a left side view of the large diametered key ring;

Fig. 6A is a front view showing a lower half part of a key ring of small diameter in cross section;

Fig. 6B is a right side view of the small diametered key ring;

Fig. 6C is a left side view of the small diametered key ring;

Fig. 7 is a left side view of the male member;

Fig. 8 is a whole view of a plasmapheresis bag set provided with a connector of the invention;

Fig. 9 is a perspective view of a disassembled connector of another embodiment of the invention;

Fig. 10 is a vertically cross sectional view of a setup connector of Fig. 9;

Fig. 11 is a front view of an upper half cross section showing another embodiment of a connecting means for male and female members;

Fig. 12 is a perspective view showing a further engaging means of said two members;

Fig. 13 is a perspective view showing a still further embodiment of the invention;

Fig. 14A is a side view of the male member of Fig. 13;

Fig. 14B is a side view of the female member of Fig. 13;

Fig. 14C is a cross sectional views along I-I and II-II of the both members of Fig. 13;

Fig. 15 is a perspective view of a disassembled male member of Fig. 13;

Fig. 16 is an outlined view of a setting-up device for the male member of Fig. 13;

Fig. 17 is a cross sectional view for explaining another setting-up manner of the male member;

Fig. 18 is an outlined view of sealing the both members with a protector;

Fig. 19 is an outlined view of applying a sealing member to a connection between the both members;

Fig. 20 is a perspective view showing another embodiment of the invention;

Fig. 21A is a side view of the connecting side of the male member of Fig. 20;

Fig. 21B is a side view of the connecting side of the female member of Fig. 20;

Fig. 21C is cross sectional views along III-III and IV-IV of the both members of Fig. 20;

Fig. 22 is an outlined view of a foregoing plasmapheresis bag set; and

Fig. 23 is an outlined view of a blood restoring set to be connected to a foregoing plasmapheresis bag set.

Description of preferred embodiments

The present invention will be explained with reference to embodiments shown in the attached drawings.

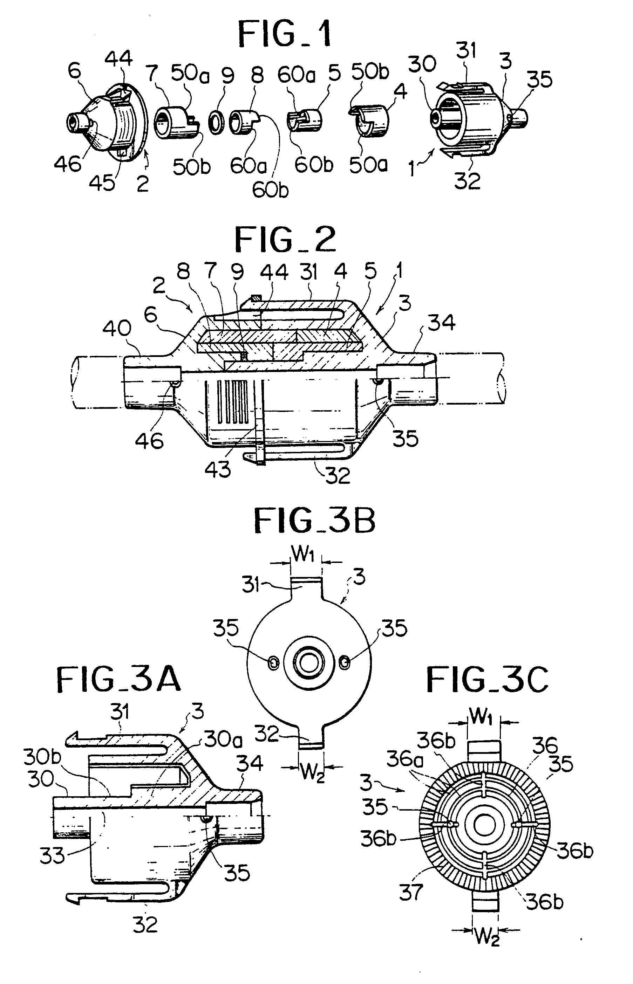

Fig. 1 is a perspective view of a disassembled connector and Fig. 2 is a front view showing an upper half part of the connector in cross section.

In the same, the numeral 1 is a first member and 2 is a second member. The first member 1 is composed of a housing 3, a key ring 4 of large diameter and a key ring 5 of small diameter. The second member 2 is also composed of a housing 6, a key ring 7 of large diameter and a key ring 8 of small diameter. Either one of the small diametered key rings 5, 8 is inserted with a seal packing 9.

Figs. 3A, B and C show in detail the housing 3 of the first member 1 which is shaped in cup as a whole and projected at a rear part, with a tube connecting mouth 34 and formed centrally with a blood path 33 as well as a male connecting tube 30 at a center of an inner part.

The male connecting tube 30 is formed, at an outer side, with a step of a large diametered part 30a at a rear part and a small diametered part 30b at a front part.

The housing 3 is formed with a pair of hooks 31, 32 at an upper and a lower part, having elasticity in vertical directions, and the hook 31 has a width W1 larger than a width W2 of the other hood 32.

In such a manner, a vertically reversed connection of the first and second members is never made, and since the hooks are provided at the two parts, the connection is secured. If thicknesses are made different in the hooks 31 and 32, said reversed connection may be avoided.

The housing 3 is formed, at its rear part, with a plurality of housing agent pouring holes 35, 35 passing to the interior of the housing, and is formed, at its inner wall, with a bonding agent guide groove 36 which is, as seen in Fig. 3C, composed of a plurality of circular grooves 36a formed at an inner wall and a plurality of grooves 36b communicating with said circular grooves 36a and extending in length of an inner wall of the housing and in length of said large diametered part 30a.

The housing is marked with scalings 37 at the end of the opening, which are each marked per, e.g. 5° circumferentially around a core of the housing.

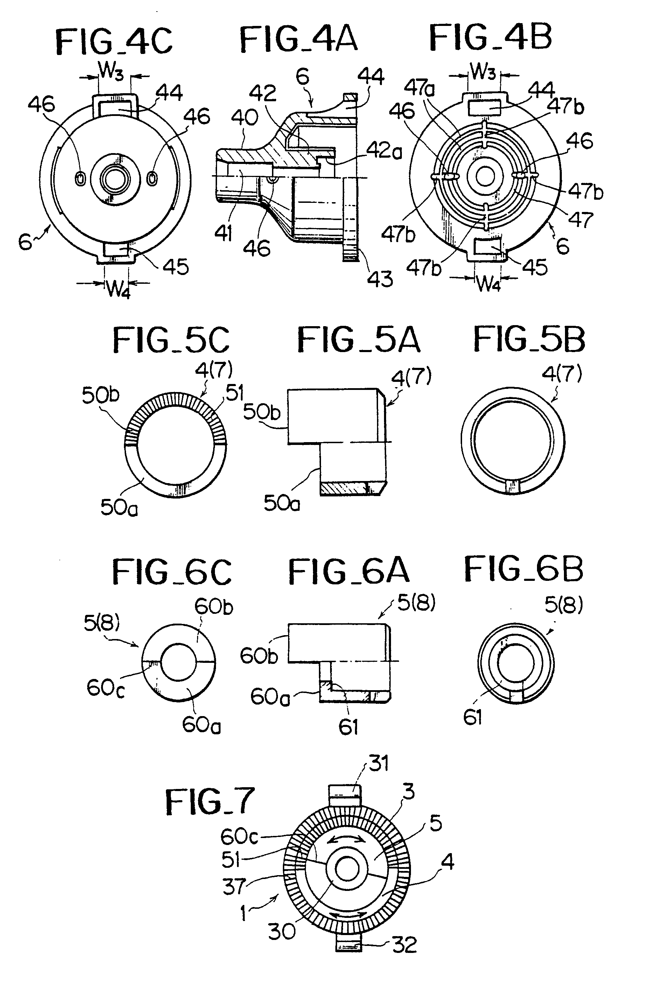

Figs. 4A, B, C show in detail a housing 6 of the second member 2 which is shaped in cup as a whole and projected at a rear part with a tube connecting mouth 40 and formed centrally with a blood path 41 as well as a female connecting tube 42 at a center of an inner part.

The female connection tube 42 is formed with a concave 42a at its end to which the end portion of the male connection tube 30 has access.

The housing 6 is formed with a flange 43 on the outer circumference in the opening side, which is opened with engaging holes 44, 45 corresponding to the hooks 31, 32 of the housing 3 of the first member as shown in Figs. 4B and 4C, and the hole 44 has a larger width W3 than W4 of the hole 45.

The housing 6 is formed similarly as mentioned above, at its rear part, with a plurality of bonding agent pouring holes 46, 46 passing to the interior of the housing, and is formed, at its inner wall, with a bonding agent guide groove 47 which communicates with said holes 46, 46, and is also composed of a circular groove 47a and a plurality of grooves 47b extending in length of an inner circumferential wall of the housing and in length of an outer face of said communication tube 42.

Figs. 5A, B, C show in detail the large diametered key rings 4, 7 which are formed with a concave 50a and a convex 50b by cutting out a half part of a circumference at an end of the tube body, and the convex 50b is marked with scalings 51 which are each marked per, e.g. 5° circumferentially around a core of the housing, as similarly the scaling 37 of the housing 3 of the first member.

Figs. 6A, B, C shows in detail the small diametered key rings 5, 8 which are formed with a concave 60a and a convex 60b by cutting out a half part of a circumference at an end of the tube body, and the convex 60a is formed with a projected flange 61. The small diametered key ring 8 is to be incorporated in the housing of the second member, and is inserted with a seal packing material 9 as shown in Fig. 1 so as to contact to said flange 61.

A further explanation will be made to one example of setting-up of the above mentioned connector.

At first, as shown in Fig. 7, the housing 3 of the first member 1 is inserted with the large diametered key ring 4 and the small diametered key ring 5, and a convex cutout 60c of the small diametered key ring 5 is met to one of the scalings 51 of the large diametered key ring 4, and one of the scalings 51 of the large diametered key ring 4 is met to one of the scalings 37 of the housing 3. If the scale is slided one by one, combinations of 72×72×72=373248 (the scalings are marked per 5°) are obtained in total.

The key rings 4, 5 are adjusted in positioning of the angular rotation, and the bonding agent is poured into the hole 35. The bonding agent flows from the guide groove 36 into between the outer circumference of the large diametered key ring 4 and the inner circumference of the housing 3, and between the inner circumference of the small diametered key ring 5 and the outer circumference of the large diametered part 30a of the male connecting tube 30. It becomes hardened as flowing so that the key rings 4, 5 are secured to the housing.

On the other hand, the second member 2 is also inserted, in the housing 6, with the large diametered key ring 7 and the small diametered key ring 8, and the rotational angle is adjusted such that the key rings 7, 8 are fitted with the key rings ket rings 4, 5 with respect to the concave and convex, and the bonding agent is poured into the holes 46, 46 of the housing 6 so as to secure the key rings 4, 5 to the housing 6 similarly as the first member 1. When the small diametered key rings 5, 8 and the large diametered key rings 4, 7 are met a tube has a double layer.

In the above embodiment, the connector comprises the large diametered key ring and the small diametered key ring, but if kinds of the combination may be reduced, either one will be omitted, and if many kinds are required other key rings will be added.

The bonding agent guiding grooves 36, 37 are not limited to the shown ones, but any types will be enough if the key rings are exactly secured.

For materials of the housings or key rings, synthetic resins excellent in suiting to human living bodies are preferable, for example, polycarbonate, polypropylene, vinyl chloride or the like are used. For the seal packing material rubber or elastic synthetic resin are used.

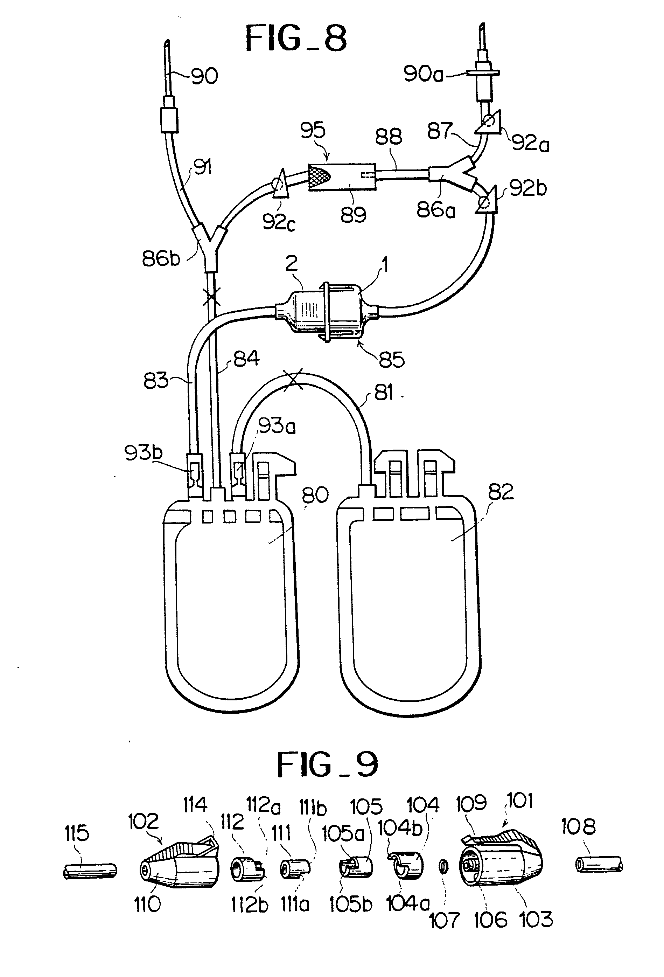

Fig. 8 shows an embodiment where the connector of the invention is applied to a plasmapheresis bag system.

A blood transfusion bag 80 is connected to a blood plasma separating bag 82 via a connection tube 81, and to a blood restoring tube 83 and a blood guiding tube 84. The blood restoring tube 83 is, on half way, connected to the connector 85 of the present invention, and a tube extending therefrom is connected to a Y-tube 86a which is connected to a tube 87 having a physiological saline solution guide needle 90a and to a tube 88 for mixing the blood and the physiological saline solution.

The mixing tube 88 is provided with an instillation tube 89, and a tube extending therefrom is connected to a Y-tube 86b which is connected to a tube 91 having a blood guiding needle 90 and said blood guiding tube 84.

In the same, numerals 92a, 92b, 92c designate flowing amount adjusting clamps, and the numerals 93a, 93b designate communication pieces.

The above plasmapheresis bag will be referred to in its usage.

When the blood drawing bag 80 is positioned above a blood donor and the needle 90 is pierced into his blood tube, the blood is guided to a blood bag 37 via the tubes 91, 84. Then, the clamps 92a, 92b, 92c are closed.

After a determined amount of the blood is guided, the tube 84 is closed with a welder and cut off, the connector 85 is divided into the first part 1 and the second part 2, and they are shielded with caps for preventing from the air.

If the bag 80 supporting the blood is subjected to a centrifugal separation together with a blood plasma separation bag 82, the blood therein is divided into the blood component and the blood plasma component.

Subsequently, the communication piece 93a is cut off to transfer a supernatant blood plasma component in the blood bag 80 to said bag 82 via the communication tube 81.

The communication tube 91 is closed with the welder, and the blood bag 80 and the blood plasma bag 81 are cut off. The blood component only remains in the blood bag 80, and this blood component is returned to the blood donor.

The needle 90a is pierced into a container (not shown) enveloping the physiological saline solution, and the clamp 92a is released opened so as to guide the saline solution in the blood returning circuit 95 for performing a priming therein, after which, the needle 90 is again pierced into his blood tube, and the second member 2 of the connector 85 provided at the blood returning tube 83 of the blood bag 80 is connected to the first member 1. Said circuit 95 is composed of the first member 1, the needle 89, the tube 87, the mixing tube 88, the instillation bag 89, the blood needle 90, and the tube 91.

The key rings 4, 5 of the first member 1 and the key rings 7, 8 of the member 2 are arranged in proper positions per each of lots so that they are connected each other. Since the housings 3, 6 are so controlled that hooks 31, 32 are engaged with the engaging holes 43, 44 and in case of the blood bag 80 of the blood donor, the member 1 and the member 2 agree to each other, otherwise the both are not met.

Therefore, by the agreement of the both members 1 and 2, an identification of the blood donor and the blood component may be checked.

If the communication piece 93b and the clamps 92b, 92c are opened after communication of the member 1 and the member 2, the blood component in the bag is guided to the blood returning circuit 95, and it is completely mixed with the saline solution and diluted, and returned to the blood donor.

The connector of the invention may be, of course, applied to (a) plasmapheresis bag provided with more than two pairs of a blood bag—a blood plasma bag, and (b) plasmapheresis bag provided with more than one pair of a blood bag—a blood plasma separating bag—a small bag, other than the plasmapheresis bag as shown in Fig. 8.

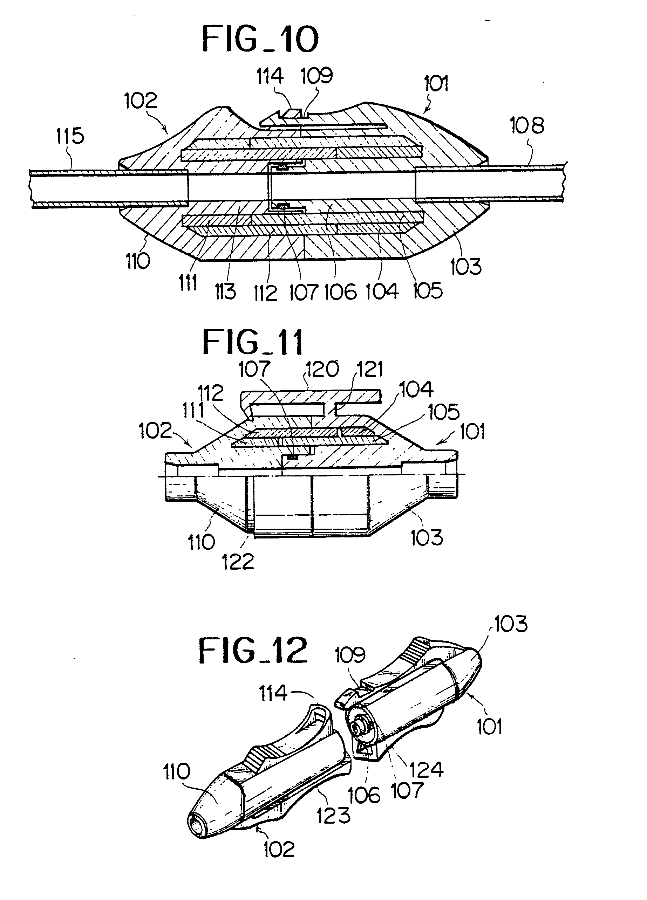

Figs. 9 and 10 show another embodiment of the invention, and the numeral 101 is a first male member and 102 is a second female member.

The male member 101 comprises a housing 103 and a pair of large and small key rings 104, 105. The housing 103 is formed at its center with a connecting tube 106 to which a seal ring 107 is mounted. The housing 103 is provided, on an outside, with a hook 109 having elasticity in vertical directions, and connected, at a rear part, to a fluid tube 108.

The large diametered key ring 104 is fixedly inserted with the small diametered key ring 105 therewithin. The key rings 104, 105 are formed with concaves 104a, 105a and convexs 104b, 105b by cutting out the half part of its end part. The small diametered key ring 105 is inserted into the large diametered key ring 104 by sliding the concave and convex in the circumferential direction per each of the products.

The female member 102 also comprises a housing 110 and a pair of large and small key rings 111, 112 similarly to the male member.

The housing 110 is formed centrally with a communicating tube 113 into which an end portion of the communicating tube 116 of the male member, and is formed outsides with an engaging part 114 at a position corresponding to a hook 109 of the housing 101 of the male side, and further connected at a rear side with a fluid tube 115 communicating with the connecting tube 113.

The large diametered key ring 112 is inserted with the small diametered key ring 111 therein, and those are fixedly secured within the connecting tube 113. The key rings 111, 112 are formed with concaves 111a, 112a and convexes 111b, 112b and those correspond to concaves and convexes of the male key rings 104, 105 and are slided in a circumferential direction. The convexes 104b, 105b of the male key rings 104, 105 are engaged with the concaves 111a, 112a of the feamel key rings 111, 112.

Fig. 11 shows a modified example of an engaging part of the housing. The engaging part is provided with a hook 120 around a fulcrum of a supporter 121, said hook 120 being elastically movable in vertical directions, and being engaged, at its end portion, with a ring shaped groove 122 of the housing 110 of the female side. The hook 120 may be formed at the upper and lower sides.

Fig. 12 shows another embodiment of an engaging part of the housing. In addition to the hook 109 and the stopper 114 shown in Figs. 9 and 10, a hook portion 123 and an engaging portion 124 are provided on the lower part of the housing in opposition facing directions. Since the key rings shown in the above embodiments have the same shapes of the male and female sides, mass-production is possible at low cost.

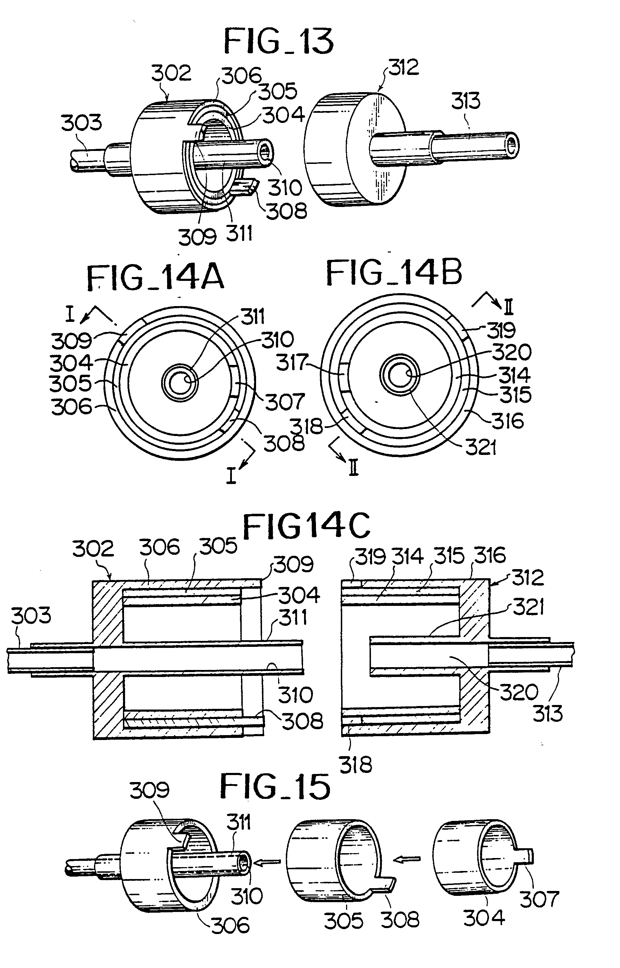

Figs. 13, 14 and 15 show further embodiments of the invention. The numeral 302 is a first member which comrises a ring 306 of cap shape and a large and small rings 304, 305, said ring 306 being a housing.

The rings 304, 305, 306 are formed with fitting projections 307, 308, 309, and the ring 306 is projected with a communication tube 311 having a fluid path 310 and is connected to a blood guide tube 303 at its end.

A second member 312 is composed of a cap shaped ring 316 to be a housing, and large and small rings 314, 315 fixed in the ring 306 in correspondence to the first member 302. These rings 314, 315, 316 are formed with fitting grooves 317, 318, 319 corresponding to projections 307, 308, 309 of the member 302.

The ring 316 is projected with a communication tube 321 having a fluid path 320 and fittable to the communication tube 311, and is connected to a blood guide tube 313 at its end.

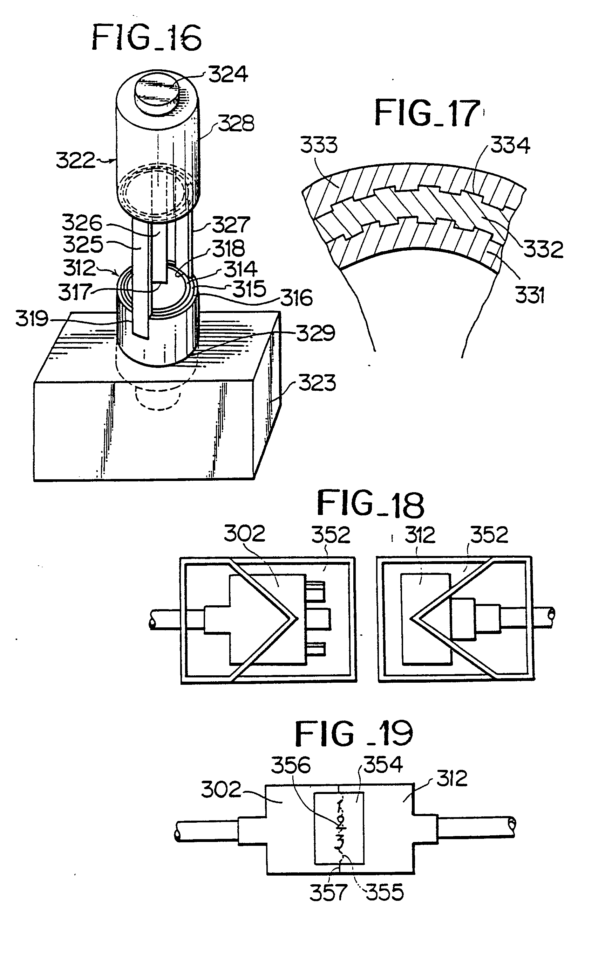

Fig. 16 schematically shows a device for setting up the second member 312. This device comprises a base 323 of the female member and a rotational angle adjusting device 322 of the ring, and the adjusting device 322 is composed of a rotational device 328 having three pins 325, 326, 327 and a drive device 324.

Each of the pins 325, 326, 327 is independently rotated by a determined angle by means of a control device incorporated ;in the rotation device 328.

The above mentioned setting-up device is actuated as under:

A ring 316 is incorporated in the connector order with the rings 315, 314 so as to make up a second member 312 of three layered structure, and this second member 312 is inserted fixedly in a concave 329 of the base 323.

Then, the device 322 is moved down to insert the pins 325, 326, 327 into the grooves 317, 318, 319. Subsequently, under a condition that, e.g., the pins 325, 326 secure the rings 314, 316, the pin 327 is rotated at a determined angle so that the position of the fitting groove 318 of the ring 315 is adjusted intentionally.

In the above manner, if the groove 318 of the ring 315 is adjusted in positioning per rotational angle 0=5°, the second members of 72 kinds different in fittings are obtained. When the positions of the fitting grooves of the rings 315, 314 are adjusted, second members different in fitting embodiments of 72×72=5184 kinds are obtained, and further when the positions of the fitting grooves of the rings 315, 314 and 316 are adjusted, second members different in fittings of 72×72×72=373248 kinds are obtained. The above mentioned refers to setting-up of the second member 312, but also with respect to the first member 302, the pins 325, 326, 327 are, at ends, provided with members for clamping the projections 304, 308, 309, and the setting-up may be carried out in the same manner as above. This setting-up is useful to setting-up of a connector having a key ring in the connecting member, and is of course applicable to the connectors as shown in Figs. 1, 9 and 17.

Fig. 17 is a partially enlarged cross sectional view showing setting-up of rings composing the first and second members. 331, 332, 333 show rings different in diameter respectively. If these rings are female, the fitting grooves are formed, and if they are male, the fitting projections are formed, through not shown. The ring 333 is formed with concave-convex 334 on its inner part, the ring 332 is formed on its inner and outer parts with them, and the ring 331 is done on its outer part, so that these rings are fitted each other. The spaces of the concave and convex are formed by, for example, each 5° in the circumferential direction. Therefore, if the rings 331, 332, 333 are incorporated in succession, as the concave and convex 334 are slided, the combinations of 373248 kinds are obtained at the maximum.

The usage of the above connector is the same as explained in Fig. 8 where the first member 302 and the seond member 312 are in advance connected, and they are separated for use. As shown in Fig. 18 the both members are separated, and are housed air-tightly the protector of soft plastic will be broken when they are used.

In such a manner, it is no longer necessary to cap the bag against the air, after the blood of a determined amount is colleted in the blood bag, and the working is very hygienic.

The connector 85 shown in Fig. 8 is in advance divided into the first and second ones, and they may be maintained air-tight.

Further in the invention, as shown in Fig. 19, a seal 354 is pasted to a connection between the first and second members for checking confirmation.

The seal 354 is centrally perforated in wave and checking letters or figures 356 are printed symmetrically at the perforations 355. The seal 354 is attached in alignment with a connecting line 357 between the first member 302 and the second member 312.

If the seal 354 is pasted on the outer circumference by sliding the position per each of the connectors, and when the member and the member are different in kind, the letters 356 and the perforations 355 are not met so that the identification could be easily seen.

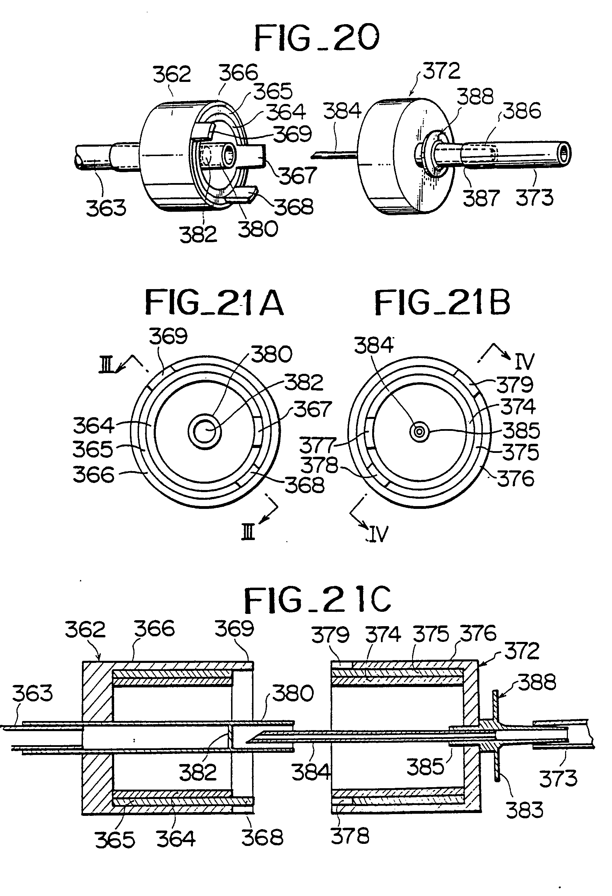

Figs. 20 and 21 show a modification of the embodiment of Figs. 13 and 14. The numeral 362 designates a first member having a three layered structure by laminating three kind rings of different diameter.

The rings 364, 365, 366 are formed with projections 367, 368, 369, and the ring 366 to be a housing is provided with a communication tube 380 which is centrally formed with a thin breakable part 382, and is connected with a blood guide tube 363 at its rear end.

The blood guide tube 363 may be extended into the ring 366 for providing the breakable part at its end.

372 is a second member having layers of three kind rings as said second member 312.

The rings 374, 375, 376 are formed with grooves 377, 378, 379 fittable to projections 367, 368, 369 of the first member 362. The ring 376 to be a housing is provided with a needle member 388 at its rear part, which is composed of a root 385 having a flange 383 and a needle tube 384 to be connected to said root 385, and the root is connected to the blood guide tube 373.

The first member 362 and the second member 372 are set up in the same process as said first and second members 302 and 372, and finally the communication tube 380 and the needle 388 are furnished.

The first member 362 anf the second member 372 are capped on the communication tube 380 and the needle member 388, and each of the members is independently protected as shown in Fig. 18, and applied to the plasmapheresis bag.

For use, the cover or cap are taken off, and the projections 367, 368, 369 of the first member 362 agree to the grooves 377, 378, 379 of the second member 372. Only when they are fitted (when the blood donor and the blood component to be restored are identified), the needle 384 of the second member 372 pierces the breakable part 382 of the communication tube 380 of the member 362 for introducing the blood to the returning circuit.

Each of the above mentioned embodiments is listed for example, and actual structure may be modified within claimed range. For instance, the composing elements of the first member are furnished to the second member, and the corresponding composing elements of the second member are furnished to the first member. Thus, the present invention provides a connector for a blood restoring or returning conduit of a plasmapheresis bag set, said connector comprising first and second connecting members. Each of these members is provided with a laminated or nested key ring structure. The key rings are formed, at their ends, with at least one convex or protruding portion and at least one groove or concave or recessed portion to provide a combination of said members. The connecting members are provided with positioning means and the positions of the different portions of the key rings are adjusted individually for each connector.

**Claims**

1. A connector for plasmapheresis bag, which is to be furnished to an induction tube of the plasmapheresis bag which restores a blood component to a blood donor, after having divided a collected blood into a blood component and a blood plasma component, and collected the blood plasma component, comprising first and second connecting members (1, 2), characterized in that:
   the first connecting member (1) is coaxially assembled with a plurality of key rings comprising tube bodies (4, 5) having projections (50b, 60b), and
   the second connecting member (2) is coaxially assembled with a plurality of key rings comrising tube bodies (7, 8) having grooves (50a, 60a) corresponding to said projections (50b, 60b),
   whereby the projections (50b, 60b) and the grooves (50a, 60a) are positioned to coincide each other when the connecting members (1, 2) are connected.

2. The connector as claimed in claim 1, wherein a casing part (3) of the first connecting member (1) is defined with hook portions (31, 32) while a further casing part (6) of the other connecting member (2) is defined with engaging portions (44, 45) corresponding to the hook portions (31, 32).

3. The connector as claimed in claim 2, wherein the hook portions (31, 32) and the engaging portions (44, 45) are plural, and width and/or thickness of each hook portion and each engaging portion are made different such that only the corresponding hook portion and engaging portion are engaged.

4. The connector as claimed in claim 1, 2 or 3, wherein each of the end portions of the tube bodies (4, 5, 7, 8) of the both connecting members (1, 2) is defined in a half cutout to form convexes (50b, 60b) and concaves (50a, 60a).

5. The connector as claimed in one of claims 1 to 4, wherein the tube bodies (304, 305, 306) of the one connecting member (302) are formed at end portions thereof with projections (307, 308, 309), while the tube bodies (304, 305, 306) of the other connecting member (312) are formed with grooves (317, 318, 319) to be engaged with said projections.

6. The connector as claimed in one of claims 1 to 5, wherein the first connecting member (1) is provided with a male tube (30) for communication with an induction tube, while the other connecting member (2) is provided with a female tube (42) for the male tube (30), communicating with the other induction tube, and said male tube (30) and said female tube (42) are connected via a packing (9).

7. The connector as claimed in one of claims 1 to 6, wherein one connecting member (372) is provided with a needle member (388) for communication with an induction tube, while the other connecting member (362) is provided with a tube (380) connecting said needle member and communicating with the other induction tube, said tube (380) being formed therewithin with a closing part (382) which is breakable by said needle member.

8. The connector as claimed in one of claims 1 to 7, wherein each of the connecting members is sealed with a protector made of soft plastics.

**Patentansprüche**

1. Verbindungseinrichtung für einen Plasmapheresebeutel, dessen Inhalt einer Einleitungsröhre des Plasmapheresebeutels zuzuführen ist, welche einen Blutbestandteil zu einem Blutspender zurückführt, nachdem gespendetes Blut in einen Blutbestandteil und einen Blutplasmabestandteil getrennt und der Blutplasmabestandteil gesammelt worden ist, enthaltend ein erstes und ein zweites Verbindungselement (1, 2), dadurch gekennzeichnet, daß
   das erste Verbindungselement (1) koaxial zusammengesetzt ist aus einer Anzahl voin Paß-ringen, die mit Vorsprüngen (50b, 60b) versehene Röhrenkörper (4, 5) aufweisen, und

das zweite Verbindungselement (2) koaxial zusammengesetzt ist aus einer Anzahl von Paßringen, die mit den Vorsprüngen (50b, 60b) entsprechenden Aussparungen (50a, 60a) versehene Röhrenkörper (7, 8) aufweisen,

wobei die Vorsprünge (50b, 60b) und die Aussparungen (50a, 60a) so angeordnet sind, daß sie miteinander übereinstimmen, wenn die Verbindungselemente (1, 2) verbunden werden.

2. Verbindungseinrichtung nach Anspruch 1, bei der ein Gehäuseteil (3) des ersten Verbindungselementes (1) mit Hakenbereichen (31, 32) versehen ist, während ein weiteres Gehäuseteil (6) das anderen Verbindungselements (2) mit den Haken-bereichen (31, 32) entsprechenden Einrastberei-chen (44, 45) versehen ist.

3. Verbindungseinrichtung nach Anspruch 2, bei der die Hakenbereiche (31, 32) und die Einrastbereiche (44, 45) in Mehrzahl vorliegen, und die Breite und/oder Dicke jedes Hakenbereichs und jedes Einrastbereichs verschieden sind, so daß nur der entsprechende Hakenbereich und Einrastbereich in Eingriff stehen.

4. Verbindungseinrichtung nach Anspruch 1, 2 oder 3, bei der jeder der Endbereiche der Röhrenkörper (4, 5, 7, 8) der beiden Verbindungselemente (1, 2) durch einen Halbausschnitt begrenzt ist, um Konvexteile (50b, 60b) und Konkavteile (50a, 60a) zu bilden.

5. Verbindungseinrichtung nach einem der Ansprüche 1 bis 4, bei der die Röhrenkörper (404, 405, 406) des einen Verbindungselements (302) an seinen Endbereichen mit Vorsprüngen (307, 308, 309) versehen ist, während die Röhrenkörper (304, 305, 306) des anderen Verbindungselements (312) mit Aussparungen (317, 318, 319) versehen ist, die mit den Vorsprüngen in Eingriff zu bringen sind.

6. Verbindungseinrichtung nach einem der Ansprüche 1 bis 5, bei der das erste Verbindungselement (1) mit einer Steckröhre (30) versehen ist, die mit einer Einleitungsröhre in Verbindung steht, während das andere Verbindungselement (2) mit einer Buchsenröhre (42) für die Steckröhre (30) versehen ist, die mit einer weiteren Einleitungsröhre in Verbindung steht, und die Steckröhre (30) und die Buchsenröhre (42) durch eine Dichtung (9) verbunden sind.

7. Verbindungseinrichtung nach einem der Ansprüche 1 bis 6, bei der das eine Verbindungselement (372) mit einem Nadelelement (388) versehen ist, das mit einer Einleitungsröhre in Verbindung steht, während das andere Verbindungselement (362) mit einer Röhre (380) zum Anschluß an das Nadelelement und mit der anderen Einleitungsröhre in Verbindung stehend versehen ist, wobei die Röhre (380) in ihrem Inneren ein von dem Nadelelement durchbrechbares Verschlußteil (382) aufweist.

8. Verbindungseinrichtung nach einem der Ansprüche 1 bis 7, bei der jedes der Verbindungselemente in einer aus Weichkunststoff gefertigten Schutzhülle eingeschlossen ist.

**Revendications**

1. Connecteur pour sac de plasmaphérèse, devant être placé dans un tube d'induction du sac de plasmaphérèse restituant un composant sanguin à un donneur de sang, après avoir séparé le sang collecté en composant sanguin et composant de plasma sanguin, et collecté le composant de plasma sanguin, comprenant des premier et deuxième organes de connexion (1, 2), caractérisé en ce que:

le premier organe de connexion (1) est assemblé coaxialement avec une pluralité de bagues d'imbrication comprenant des corps tubulaires (4, 5) munis de saillies (50b, 60b), et

le second organe de connexion (2) est assemblé coaxialement avec une pluralité de bagues d'imbrication comprenant des corps tubulaires (7, 8) présentant des gorges (50a, 60a) correspondant auxdites saillies (50b, 60b),

et en ce que les saillies (50b, 60b) et les gorges (50a, 60a) sont positionnées pour coïncider les unes avec les autres lorsque les organes de connexion (1, 2) sont connectés.

2. Connecteur selon la revendication 1, dans lequel une partie de boîtier (3) du premier organe de connexion (1) est définie avec des parties en crochet (31, 32) tandis qu'une autre partie de boîtier (6) de l'autre organe de connexion (2) est définie avec des parties d'engagement (44, 45) correspondant aux parties en crochet (31, 32).

3. Connecteur selon la revendication 2, dans lequel les parties en crochet (31, 32) et les parties d'engagement (44, 45) sont multiples et la largeur et/ou l'épaisseur de chaque partie en crochet et de chaque partie d'engagement sont différentes de façon que seules la partie en crochet et la partie d'engagement correspondantes sont en prise.

4. Connecteur selon l'une des revendications 1, 2 ou 3, dans lequel chacune des parties d'extrémité des corps tubulaires (4, 5, 7, 8) des deux organes de connexion (1, 2) est définie sous forme d'une découpe en demi-pièce, afin de donner des formes convexes (50b, 60b) et concaves (50a, 60a).

5. Connecteur selon l'une quelconque des revendications 1 à 4, dans lequel les corps tubulaires (304, 305, 306) d'un organe de connexion (302) sont formés sur ses parties d'extrémité, avec des saillies (307, 308, 309), tandis que les corps tubulaires (304, 305, 306) de l'autre organe de connexion (312) sont formés avec des gorges (317, 318, 319) devant venir au contact desdites saillies.

6. Connecteur selon l'une quelconque des revendications 1 à 5, dans lequel le premier organe de connexion (1) est pourvu d'un tube mâle (30), pour communiquer avec un tube d'induction, tandis que l'autre organe de connexion (2) est pourvu d'un tube femelle (42) pour le tube mâle (30), communiquant avec l'autre tube d'induction, et en ce que ledit tube mâle (30) et ledit tube femelle (42) sont reliés par l'intermédiaire d'une garniture d'étanchéité (9).

7. Connecteur selon l'une quelconque des revendications 1 à 6, dans lequel un organe de connexion (372) est pourvu d'un organe en aiguille (388) pour communiquer avec un tube d'induction, tandis que l'autre organe de connexion (362) est pourvu d'un tube (380) reliant ledit organe en aiguille et le faisant communiquer avec l'autre tube à induction, ledit tube (380) étant formé à l'intérieur, avec une partie de fermeture (382) pouvant être fracturée par ledit organe en aiguille.

8. Connecteur selon l'une quelconque des revendications 1 à 7, dans lequel chaque organe de connexion est isolé de manière étanche à l'aide d'un protecteur fait d'une matière plastique souple.

# FIG_1

# FIG_2

# FIG_3B

# FIG_3A

# FIG_3C

FIG_4C

FIG_4A

FIG_4B

FIG_5C

FIG_5A

FIG_5B

FIG_6C

FIG_6A

FIG_6B

FIG_7

2

# FIG_8

90
90a
95
88
87
92a
91
92c
89
86a
92b
86b
2
1
85
83
84
81
93a
93b
80
82

# FIG_9

115
102
114
112
112a
111b
105
104
109
101
108
110
112b
111
111a
105a
105b
104b
104a
107
106
103

## FIG_10

## FIG_11

## FIG_12

FIG_13

FIG_14A

FIG_14B

FIG14C

FIG_15

FIG_16

324
328
322
326
327
325
318
312
314
317
315 316
319
329
323

FIG_17

333
334
332
331

FIG_18

302 352 312 352

FIG_19

356 354 312
302

357 355

## FIG_20

362 366
365
364
369
372 388 386
384
367
363
382 380
387 373

## FIG_21A

369
380
382
367
364
365
366
368

II
II

## FIG_21B

384
IV
379
374
385
375
376
377
378
IV

## FIG_21C

362 366 369
379 374 375 376
372
388
363
380
382
384
385
373
383
365 364 368
378

# FIG_22
(PRIOR ART)

# FIG_23
(PRIOR ART)